# FASCICULE DE BREVET EUROPEEN

(11) **EP 2 007 270 B1**
(45) Date de publication et mention de la délivrance du brevet: **07.08.2019**
(21) Numéro de dépôt: 07731242.9
(22) Date de dépôt: 03.04.2007
(51) Int. Cl.: A61B 1/04, A61B 1/07

(54) **PROTECTION POUR ENDOSCOPE ET ENDOSCOPE CORRESPONDANT**
SCHUTZ FÜR EIN ENDOSKOP UND ENTSPRECHENDES ENDOSKOP
PROTECTION FOR ENDOSCOPE, AND CORRESPONDING ENDOSCOPE

(30) Priorité: 03.04.2006 FR 0602887
(43) Date de publication de la demande: 31.12.2008
(73) Titulaire: V.I.M.S. Video Interventionnelle Médicale Scientifique, 31620 Villeneuve-lès-Bouloc (FR)
(72) Inventeur: FERNANDEZ, Henri, F-31330 Larra (FR); RODDIER, Nicolas, F-31750 Escalquens (FR); BRUNEL, Lin, F-31700 Blagnac (FR)
(74) Mandataire: Santarelli
(86) Numéro de dépôt international: PCT/FR2007/000564
(87) Numéro de publication internationale: WO 2007/113400

(56) Documents cités:
- EP-A1- 0 211 976
- EP-A1- 0 456 761
- EP-A1- 0 904 725
- US-A- 3 801 181
- US-A- 5 377 047
- US-A- 5 704 892
- US-A- 6 124 883
- US-A1- 2003 216 618

## Description

La présente invention concerne une protection pour endoscope ainsi qu'un endoscope correspondant.

Un endoscope est un instrument utilisé en médecine. Cet instrument comporte un système optique pour examiner la surface interne d'un organe creux, d'une cavité naturelle ou d'un conduit du corps à des fins diagnostiques ou thérapeutiques. Un tel instrument comporte une partie invasive appelée tige d'endoscope, pénétrant dans le corps d'un patient, et des moyens associés, tels par exemple un oeilleton, permettant de voir à l'intérieur du corps du patient. Une source lumineuse permettant d'éclairer la surface à observer est aussi généralement associée à l'endoscope.

Un endoscope rigide, dont il est question dans le présent document, présente une partie invasive rigide par opposition aux endoscopes flexibles appelés aussi parfois fibroscope. La partie invasive d'un endoscope rigide présente habituellement une forme cylindrique circulaire d'un diamètre de 8 mm par exemple. Elle comporte une enveloppe métallique tubulaire cylindrique contenant d'une part des lentilles optiques permettant d'observer depuis un oeilleton ou similaire la surface interne du corps à examiner et d'autre part des fibres optiques destinées à conduire de la lumière depuis une source lumineuse externe pour éclairer la surface à examiner.

L'endoscope peut être associé à un capteur d'images, tel par exemple une caméra. L'appareil alors obtenu est un endoscope appelé aussi vidéo endoscope.

Pour éviter toute contamination, il est connu de stériliser les endoscopes avant chaque usage. Cette stérilisation se fait en autoclave. Le temps nécessaire pour réaliser une stérilisation d'un endoscope est important et les conditions sévères dans lesquelles cette stérilisation est réalisée conduisent à la longue à une détérioration de l'endoscope.

Le brevet EP-0 456 761 propose une gaine stérile associée à un manchon repliée en accordéon afin de recouvrir un vidéo endoscope et pouvoir éviter ainsi de devoir le stériliser. La gaine vient protéger la partie invasive de l'endoscope et le manchon est destiné à recouvrir la partie extérieure du vidéo endoscope. L'utilisation d'une telle gaine et d'un tel manchon permet de résoudre les problèmes liés à la stérilisation d'un endoscope. Toutefois la gaine recouvrant la partie invasive de l'endoscope augmente le diamètre total de l'ensemble à introduire dans le corps du patient.

La présente invention a alors pour but de remédier à ce dernier problème. Ainsi, le but de l'invention est de fournir les moyens d'avoir un ensemble comprenant une partie invasive incluant une gaine de protection stérile d'un diamètre extérieur de diamètre réduit, de préférence du même diamètre que la partie invasive d'un endoscope stérilisable et utilisé sans gaine protectrice.

A cet effet, elle propose une protection pour endoscope comportant une gaine avec une partie tubulaire cylindrique rigide et un manchon souple associé.

Selon l'invention, la partie tubulaire cylindrique rigide est réalisée dans une matière permettant le transport de la lumière ; la partie tubulaire cylindrique rigide est traitée de manière à guider la lumière en provenance d'une source de lumière de son extrémité proximale vers son extrémité distale, et l'extrémité distale de la partie tubulaire cylindrique présente des moyens permettant de diffuser et/ou d'orienter de la lumière guidée par la partie tubulaire cylindrique.

Alors que dans les dispositifs de protection de l'art antérieur le dispositif de protection n'a qu'un rôle passif, un dispositif de protection selon l'invention peut avoir un rôle actif pour l'endoscope qu'il protège en conduisant de la lumière jusqu'à la zone à examiner par l'endoscope dans le corps d'un patient. Il devient alors inutile de prévoir au niveau de l'endoscope de guider la lumière depuis son extrémité proximale vers son extrémité distale et celui-ci peut donc présenter une partie invasive rigide de diamètre moindre, destinée uniquement à transporter l'image à observer de l'extrémité distale vers l'extrémité proximale. Globalement, le diamètre extérieur d'une partie tubulaire cylindrique d'un dispositif de protection selon l'invention est sensiblement identique à celui de la partie invasive d'un endoscope de l'art antérieur sans gaine protectrice.

Pour réaliser le guidage de la lumière dans la partie tubulaire cylindrique, cette dernière présente par exemple un coeur réalisé dans un premier matériau, et le coeur de cette partie tubulaire cylindrique est recouvert tant sur sa face intérieure que sur sa face extérieure d'un revêtement réalisé dans un matériau présentant un indice de réfraction inférieur à celui du premier matériau. Dans une telle forme de réalisation, le matériau utilisé pour réaliser le coeur de la partie tubulaire cylindrique est par exemple un PMMA (polyméthylméthacrylate) ou un polystyrène tandis que les revêtements sont par exemple réalisés en PMMA ou en polymère fluoré.

Dans un dispositif de protection pour endoscope selon l'invention, les moyens permettant de diffuser et/ou d'orienter la lumière comportent par exemple une entretoise de diffusion et une lentille de correction angulaire. L'entretoise de diffusion est, dans une forme de réalisation préférée permettant une bonne diffusion de la lumière en provenance de l'extrémité proximale de la partie tubulaire cylindrique, une pièce tubulaire présentant une face transversale plane destinée à reposer contre l'extrémité distale de la partie tubulaire cylindrique tandis que la face opposée à la face transversale présente plusieurs arêtes, éventuellement arrondies, de manière à forme plusieurs prismes. Cette entretoise de diffusion peut être disposée entre la lentille de correction angulaire et l'extrémité distale de la partie tubulaire cylindrique, et un espace libre peut subsister entre l'entretoise de diffusion et la lentille de correction angulaire de manière à former une lentille d'air entre ces deux éléments. Une telle lentille d'air participe aussi à la bonne diffusion de la lumière.

La présente invention concerne également une protection pour endoscope telle que décrite plus haut et dont le diamètre extérieur de la partie tubulaire rigide est inférieur à 5 mm.

La présente invention concerne également un endoscope comportant une partie invasive présentant une extrémité proximale et une extrémité distale.

Selon l'invention, la partie invasive comporte d'une part une tige rigide avec des moyens permettant de transporter une image de sa partie distale à sa partie proximale et d'autre part un dispositif de protection tel que décrit plus haut et l'endoscope présente en outre une sortie de lumière permettant de former à l'aide de moyens d'éclairage un faisceau lumineux annulaire autour de l'extrémité proximale de la tige rigide ainsi que des moyens de fixation pour recevoir le dispositif de protection.

Une forme de réalisation d'un tel endoscope prévoit que celui-ci est un vidéo endoscope qui comporte un boîtier dans lequel se trouvent un capteur d'images et un objectif optique associé au capteur d'images, que le capteur d'images est monté coaxialement avec un guide de lumière conique disposé à l'avant de l'objectif optique de telle sorte à pouvoir diriger de la lumière issue de moyens d'éclairage extérieurs pour réaliser le faisceau annulaire lumineux.

Selon une forme de réalisation préférée d'un endoscope selon l'invention, la tige rigide est constituée d'un tube externe métallique à l'intérieur duquel est disposé un barreau optique destiné à transmettre une image depuis l'extrémité distale de la tige vers son extrémité proximale.

Des détails et avantages de la présente invention ressortiront mieux de la description qui suit, faite en référence aux dessins schématiques annexés sur lesquels :
La figure 1 est une vue d'ensemble d'un vidéo endoscope et d'une gaine selon l'invention, la gaine ne comportant pas sur cette figure de manchon souple,
La figure 2 est une vue éclatée d'une gaine, sans son manchon, selon l'invention,
La figure 3 est une vue éclatée d'un corps d'un vidéo endoscope selon l'invention,
La figure 4 montre le corps du vidéo endoscope monté,
La figure 5 montre la fixation d'un manchon sur une gaine selon l'invention,
La figure 6 est une vue à échelle agrandie d'une forme de réalisation de moyens permettant de diffuser et d'orienter de la lumière à l'extrémité de la gaine de la figure 2, et
La figure 7 est une vue en coupe longitudinale partielle d'une partie rigide d'une gaine selon l'invention.

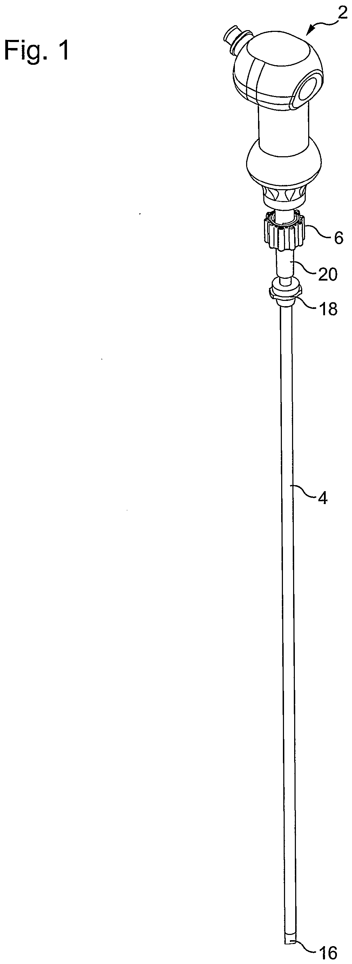

La figure 1 représente un vidéo endoscope comportant une partie invasive formée d'une tige optique rigide (non visible) recouverte par une gaine protectrice 4. Cette dernière est fixée sur l'endoscope à l'aide de moyens de verrouillage 6.

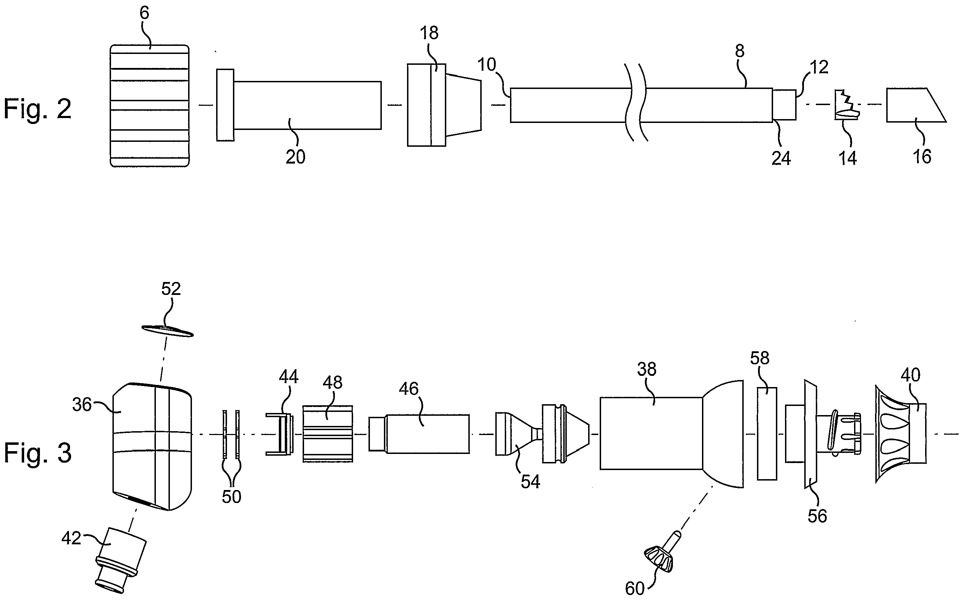

La figure 2 montre plus en détail la gaine protectrice 4. Cette dernière comporte tout d'abord une partie tubulaire cylindrique 8. Cette dernière présente une extrémité proximale 10 et une extrémité distale 12. Du côté de l'extrémité distale, la gaine protectrice 4 comporte une entretoise de diffusion 14 et une lentille de correction angulaire 16. Du côté de l'extrémité proximale 10, la gaine protectrice 4 comporte un système de blocage 18, un support de manchon 20 ainsi que les moyens de verrouillage 6.

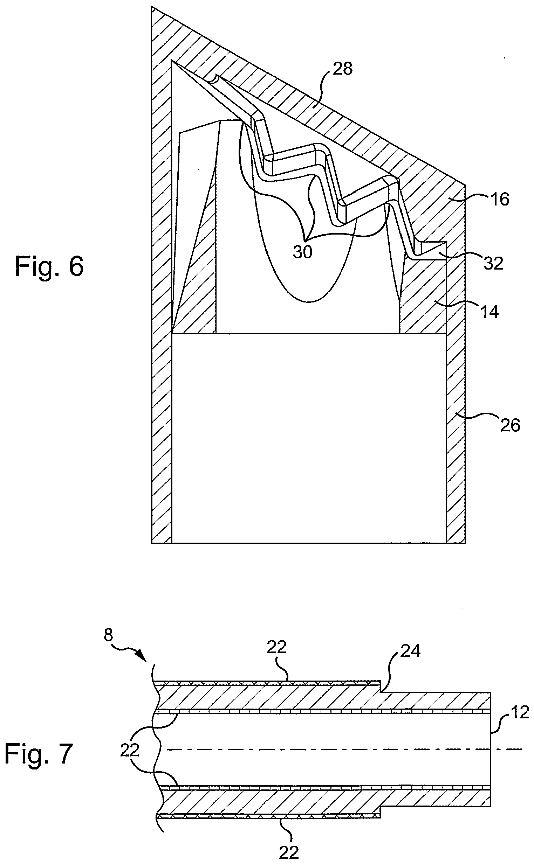

La partie tubulaire cylindrique 8 de la gaine protectrice 4 est représentée plus en détail sur la figure 7. Cette figure est une vue en coupe longitudinale de l'extrémité distale de cette partie tubulaire cylindrique 8. Le coeur de cette partie tubulaire cylindrique 8 est par exemple réalisée en polycarbonate, par exemple du PMMA (polymethylmethacrylate). Ce coeur est recouvert tant sur sa face intérieure que sur sa face extérieure d'une couche 22 réalisée dans un matériau présentant un indice de réfraction inférieur à celui du matériau utilisé pour réaliser le coeur de la partie tubulaire cylindrique 8. Il s'agit par exemple d'un polymère fluoré. Ces couches 22 peuvent être coextrudées avec le coeur lors de la fabrication de la partie tubulaire cylindrique 8 ou bien il peut également s'agir de dépôts réalisés sur et dans le coeur de la partie tubulaire cylindrique 8.

L'extrémité distale 12 de la partie tubulaire cylindrique 8 est usinée sur sa surface extérieure de manière à réaliser un épaulement 24. Ce dernier sert au montage de la lentille de correction angulaire 16. L'entretoise de diffusion 14 est montée à l'intérieur de la lentille de correction angulaire 16, entre l'extrémité distale 12 de la partie tubulaire cylindrique 8 et la lentille de correction angulaire 16. L'ensemble formé par la lentille de correction angulaire 16 et l'entretoise de diffusion 14 est montrée à échelle agrandie sur la figure 6. La lentille de correction angulaire 16 comporte d'une part une douille 26 tubulaire cylindrique et d'autre part une partie terminale 28 fermant la douille 26.

La douille 26 est de dimension adaptée à l'extrémité distale de la partie tubulaire cylindrique 8. Le diamètre intérieur de cette douille correspond au diamètre extérieur, réduit, au-delà de l'épaulement 24 de la partie tubulaire cylindrique 8. La douille 26 peut ainsi venir en butée contre l'épaulement 24.

La partie terminale 28 de la lentille de correction angulaire 16 présente une forme dépendant des caractéristiques souhaitées et de la forme de l'extrémité distale de la tige optique protégée. En effet, selon le type d'utilisation de l'endoscope, il est peut être demandé d'éclairer axialement, ou bien avec un angle donné pouvant aller jusqu'à 90°. L'exemple des dessins considère un éclairage réalisé à un angle de 30°. Ainsi, la surface extérieure de la partie terminale 28 de la lentille de correction angulaire 16 est une surface sensiblement plane inclinée de 30° par rapport à un plan transversal. La lentille de correction angulaire 16 forme ainsi un couvercle venant fermer l'extrémité distale 12 de la partie tubulaire cylindrique 8.

La lentille de correction angulaire 16 vient enfermer l'entretoise de diffusion 14 à l'extrémité distale de la gaine protectrice 4. Cette entretoise de diffusion 14 est une pièce tubulaire destinée à diffuser de la lumière guidée par la partie tubulaire cylindrique 8. Lorsque cette entretoise de diffusion 14 est en place à l'extrémité de la gaine protectrice 4, elle vient en appui contre l'extrémité distale 12 de la partie tubulaire cylindrique 8, plus précisément du coeur de cette partie. Elle présente ainsi une face annulaire plane venant en appui contre l'extrémité distale 12 de la partie tubulaire cylindrique 8. L'extrémité opposée de l'entretoise de diffusion 14 est d'une forme complexe qui dépend de l'application souhaitée pour l'endoscope, notamment de l'angle d'éclairage choisi pour orienter la lumière en provenance de l'endoscope. Comme on peut le voir notamment sur la figure 6, l'extrémité distale de l'entretoise de diffusion 14 présente des arêtes sensiblement radiales qui définissent des prismes 30. La face intérieure de la partie terminale 28 de la lentille de correction angulaire 16 reprend une forme qui correspond sensiblement à la forme de l'extrémité distale de l'entretoise de diffusion 14. Comme on peut toutefois le remarquer sur la figure 6, il subsiste entre l'entretoise de diffusion 14 et la partie terminale 28 de la lentille de correction angulaire 16 un espace libre. Seules quelques zones de contact sont prévues entre l'entretoise de diffusion 14 et la face intérieure de la partie terminale 28 de la lentille de correction angulaire 16. Ces zones de contact permettent de garantir un bon contact entre l'entretoise de diffusion 14 et la partie tubulaire cylindrique 8. On définit ainsi une lentille d'air 32 qui participe également à la diffusion et à l'orientation de la lumière.

Le système de blocage 18 se trouvant du côté de l'extrémité proximale 10 de la partie tubulaire cylindrique 8 est connu de l'homme du métier. Il s'agit d'un système que l'on retrouve classiquement sur un endoscope. En effet, un endoscope est habituellement guidé et supporté par une chemise de guidage (non représentée) et verrouillé sur celle-ci. Le système de blocage 18 correspond ici à un système de blocage connu de l'art antérieur.

De même, les moyens de verrouillage 6 permettant de fixer la gaine protectrice 4 sur l'endoscope correspondent à des moyens de verrouillage connus de l'homme du métier. Il peut s'agir par exemple de moyens de verrouillage tels ceux révélés par le document EP-0 456 761.

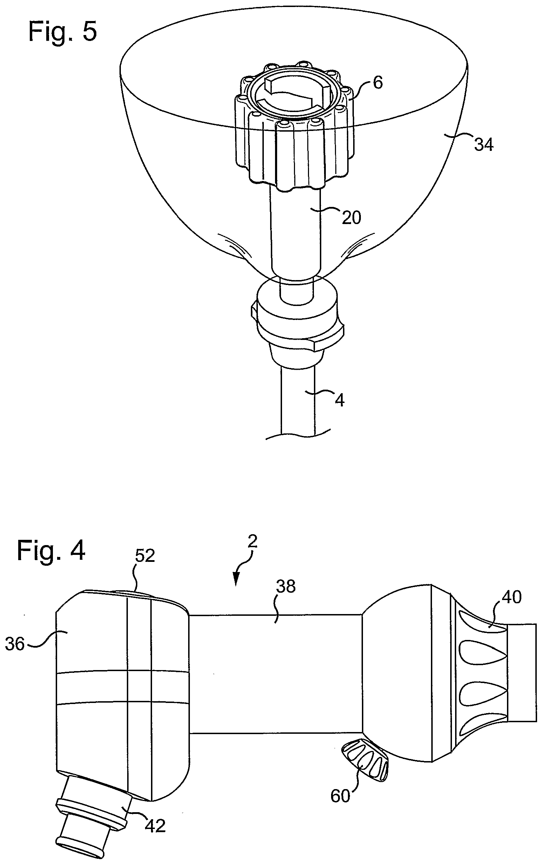

Le support de manchon 20 permet de relier un manchon souple 34 à la gaine protectrice 4 rigide (cf. figure 5). Il s'agit ici d'un manchon en matière synthétique souple. Ce manchon présente une longueur de plusieurs mètres. Ainsi, avant utilisation du dispositif de protection décrit ici, ce manchon souple 34 est replié. Pour faciliter le déploiement de ce manchon au-dessus du vidéo endoscope et de l'alimentation de cet endoscope, le manchon est de préférence replié de manière télescopique.

La figure 3 montre sous forme éclatée la poignée du vidéo endoscope et des principaux éléments qu'il contient.

Le boîtier 2 proprement dit comporte un corps arrière 36, un corps avant 38 et un capot avant 40. L'alimentation en électricité et en lumière est réalisée à travers un porte manchon 42.

A l'intérieur du boîtier 2, se trouve notamment un capteur d'images 44 associé à un objectif 46 comportant des lentilles optiques non représentées.

Le capteur d'images 44 est monté dans un support 48. A l'arrière du capteur d'images 44 se trouvent des circuits imprimés 50 intégrant des moyens électroniques pour la gestion et la commande du vidéo endoscope. Un clavier 52 fixé sur le corps arrière 36 sert d'interface entre les circuits imprimés 50 et un utilisateur.

Le vidéo endoscope est alimenté en lumière depuis une source lumineuse extérieure à travers le porte manchon 42. Ainsi, des fibres optiques alimentent le vidéo endoscope. Elles sont réparties au niveau du support 48 autour du capteur d'images 44 et de l'objectif 46 et sont ensuite connectées à un guide conique 54 de manière à former une sortie lumineuse annulaire dont les dimensions correspondent sensiblement à celles de la section de la partie tubulaire cylindrique 8 de la gaine protectrice 4. Une entretoise (non représentée) tubulaire cylindrique permet de guider la lumière depuis la sortie lumineuse annulaire à l'intérieur du boîtier 2 jusqu'aux moyens de verrouillage 6 de manière à pouvoir transmettre la lumière de la source lumineuse extérieure jusqu'à la partie tubulaire cylindrique 8 de la gaine protectrice 4 en formant alors un faisceau lumineux annulaire autour de l'extrémité proximale de la tige rigide optique.

A l'avant du boîtier 2 du vidéo endoscope, se trouve une pièce d'accouplement 56 qui porte d'une part la tige rigide optique (non représentée) et d'autre part les moyens de verrouillage complémentaires des moyens de verrouillage 6 de la gaine protectrice 4. Le capot avant 40 participe quant à lui au verrouillage de la tige rigide optique sur le vidéo endoscope.

Un roulement à billes 58 est disposé entre la pièce d'accouplement 56 et le corps avant 38 du boîtier 2. Ainsi, il est possible de faire tourner le corps du boîtier 2 avec le capteur d'images 44 par rapport à la tige optique rigide. Une vis de blocage 60 permet de bloquer le boîtier 2 dans une position donnée.

La tige rigide optique d'un endoscope selon l'invention comporte avantageusement uniquement un barreau optique disposé dans un tube, par exemple un tube métallique. Le barreau optique permet de conduire la lumière de la partie distale de la tige d'endoscope à sa partie proximale pour y être ensuite dirigé vers le capteur d'images 44. Le diamètre extérieur de cette tige d'endoscope (barreau + tube) peut être de l'ordre de 2 mm (ou même inférieur).

Dans l'ensemble décrit ci-dessus, comportant le vidéo endoscope et son dispositif de protection, la gaine protectrice 4 est une pièce active qui conduit la lumière pour éclairer l'organe à examiner par le vidéo endoscope et fait donc partie de l'endoscope. La gaine protectrice 4 et le manchon souple 34 associés ne servent donc plus ici uniquement à protéger le patient d'une contamination mais jouent également un rôle dans le fonctionnement du vidéo endoscope. Alors que dans les endoscopes de l'art antérieur, la partie invasive de l'endoscope, utilisée ou non avec une gaine stérile extérieure, sert d'une part à amener de la lumière vers la zone à examiner et d'autre part à permettre de filmer cette zone, la tige rigide optique de l'endoscope a ici pour fonction uniquement de permettre la capture d'images par le capteur d'images 44. La fonction concernant le transport de lumière vers la partie distale est entièrement dédiée à la gaine protectrice. De ce fait, le diamètre extérieur de la tige rigide optique peut être réduit. Il est possible de réaliser alors des endoscopes avec une tige optique d'un diamètre de 2 mm, voire même inférieur. La gaine protectrice 4 d'un dispositif de protection selon l'invention peut alors présenter un diamètre extérieur de l'ordre de 4 mm. Ce diamètre correspond au diamètre extérieur de la partie invasive d'un endoscope de faible diamètre de l'art antérieur. Bien entendu, l'invention peut être utilisée pour d'autres diamètres, plus ou moins importants.

Le dispositif de protection, en particulier sa gaine protectrice, permet d'assurer un bon guidage de la lumière. Par rapport aux endoscopes de l'art antérieur, une source lumineuse de puissance moindre peut être utilisée. Un endoscope tel que décrit ci-dessus peut fonctionner avec une lampe d'une puissance de 24 W alors que dans les endoscopes de l'art antérieur les lampes utilisées présentent couramment des puissances de l'ordre de 250 à 300 W.

En adaptant la forme de la lentille distale, et de l'entretoise de diffusion associée, il est possible de s'adapter à un endoscope pour tous les angles d'observations utilisés, par exemple 0°, 30°, 45°, 70° et 90°.

Dans un endoscope selon la présente invention, le dispositif de protection est à usage unique. Il peut être facilement adapté sur la tige optique rigide de l'endoscope et en être facilement retiré. La tige optique rigide est réutilisable. Entre deux interventions successives, le dispositif de protection est changé et la tige optique rigide est éventuellement désinfectée à l'aide d'un produit désinfectant. Ainsi on a un temps d'immobilisation de l'endoscope très réduit entre deux interventions.

Par rapport aux gaines protectrices de l'art antérieur qui sont utilisées, une gaine protectrice 4 selon l'invention présente une épaisseur de paroi plus importante afin de permettre de guider la lumière. Cette épaisseur accrue conduit à un gain de rigidité qui permet d'augmenter également la sécurité lors d'une intervention.

Il ressort également de ce qui précède une simplification de l'endoscope. Le coût de celui-ci peut ainsi être réduit.

La présente invention ne se limite pas à la forme de réalisation décrite ci-dessus à titre d'exemple non limitatif. Elle concerne également toutes les variantes de réalisation à la portée de l'homme du métier dans le cadre des revendications ci-après.

## Revendications

1. Protection pour endoscope comportant une gaine avec une partie tubulaire cylindrique (8) rigide et un manchon souple (34) associé,
**caractérisée en ce que** la partie tubulaire cylindrique (8) rigide est réalisée dans une matière permettant le transport de la lumière, **en ce que** la partie tubulaire cylindrique (8) rigide est traitée de manière à guider la lumière en provenance d'une source de lumière de son extrémité proximale (10) vers son extrémité distale (12), et **en ce que** l'extrémité distale (12) de la partie tubulaire cylindrique (8) présente des moyens (14, 16) permettant de diffuser et/ou d'orienter de la lumière guidée par la partie tubulaire cylindrique (8).

2. Protection pour endoscope selon la revendication 1, **caractérisée en ce que** la partie tubulaire cylindrique (8) présente un coeur réalisé dans un premier matériau, et **en ce que** le coeur de la partie tubulaire cylindrique est recouvert tant sur sa face intérieure que sur sa face extérieure d'un revêtement réalisé dans un matériau présentant un indice de réfraction inférieur à celui du premier matériau.

3. Protection pour endoscope selon la revendication 2, **caractérisée en ce que** le matériau utilisé pour réaliser le coeur de la partie tubulaire cylindrique (8) est choisi dans un ensemble comprenant les polystyrènes et les PMMA (polyméthylméthacrylate).

4. Protection pour endoscope selon la revendication 2 ou 3, **caractérisée en ce que** le matériau utilisé pour recouvrir le coeur est choisi dans l'ensemble comprenant les PMMA (polyméthylméthacrylate) et les polymères fluorés.

5. Protection pour endoscope selon l'une des revendications 1 à 4, **caractérisée en ce que** les moyens permettant de diffuser et/ou d'orienter la lumière comportent une entretoise de diffusion (14) et une lentille de correction angulaire (16).

6. Protection pour endoscope selon la revendication 5, **caractérisée en ce que** l'entretoise de diffusion (14) est une pièce tubulaire présentant une face transversale plane destinée à reposer contre l'extrémité distale (12) de la partie tubulaire cylindrique (8) tandis que la face opposée à la face transversale présente plusieurs arêtes, éventuellement arrondies, de manière à forme plusieurs prismes (30).

7. Protection pour endoscope selon l'une des revendications 5 ou 6, **caractérisée en ce que** l'entretoise de diffusion (14) est disposée entre la lentille de correction angulaire (16) et l'extrémité distale (12) de la partie tubulaire cylindrique (8), et **en ce qu'**un espace libre subsiste entre l'entretoise de diffusion (14) et la lentille de correction angulaire (16) de manière à former une lentille d'air (32) entre ces deux éléments.

8. Protection pour endoscope selon l'une des revendications 1 à 7, **caractérisé en ce que** le diamètre extérieur de sa partie tubulaire rigide est inférieur à 5 mm.

9. Endoscope comportant une partie invasive présentant une extrémité proximale et une extrémité distale,
**caractérisé en ce que** la partie invasive comporte d'une part une tige rigide avec des moyens permettant de transporter une image de sa partie distale à sa partie proximale et d'autre part un dispositif de protection selon l'une des revendications 1 à 8, et **en ce que** l'endoscope présente en outre une sortie de lumière permettant de former à l'aide de moyens d'éclairage un faisceau lumineux annulaire autour de l'extrémité proximale de la tige rigide ainsi que des moyens de fixation pour recevoir le dispositif de protection.

10. Endoscope selon la revendication 9, **caractérisé en ce qu'**il consiste en un vidéo endoscope, **en ce qu'**il comporte un boîtier (2) dans lequel se trouvent un capteur d'images (44) et un objectif optique (46) associé au capteur d'images (44), **en ce que** le capteur d'images (44) est monté coaxialement avec un guide de lumière conique (54) disposé à l'avant de l'objectif optique (46) de telle sorte à pouvoir diriger de la lumière issue de moyens d'éclairage extérieurs pour réaliser le faisceau annulaire lumineux.

11. Endoscope selon l'une des revendications 9 ou 10, **caractérisé en ce que** la tige rigide est constituée d'un tube externe métallique à l'intérieur duquel est disposé un barreau optique destiné à transmettre une image depuis l'extrémité distale de la tige vers son extrémité proximale.

## Patentansprüche

1. Schutz für ein Endoskop, enthaltend eine Hülle mit einem starren zylinderrohrförmigen Teil (8) und einen zugehörigen nachgiebigen Stutzen (34), **dadurch gekennzeichnet, dass** der starre zylinderrohrförmige Teil (8) aus einem Material hergestellt ist, das den Transport von Licht gestattet, dass der starre zylinderrohrförmige Teil (8) so ausgebildet ist, dass er das von einer Lichtquelle ausgehende Licht von seinem proximalen Ende (10) zu seinem distalen Ende (12) leitet, und dass das distale Ende (12) des zylinderrohrförmigen Teils (8) Einrichtungen (14, 16) aufweist, die das Streuen und/oder Ausrichten des Lichts gestatten, das über den zylinderrohrförmigen Teil (8) geleitet wird.

2. Schutz für ein Endoskop nach Anspruch 1, **dadurch gekennzeichnet, dass** der zylinderrohrförmige Teil (9) einen aus einem ersten Material hergestellten Kern aufweist und dass der Kern des zylinderrohrförmigen Teils sowohl auf seiner Innenseite als auch auf seiner Außenseite mit einer Beschichtung überdeckt ist, die aus einem Material herstellt ist, das einen geringeren Brechungsindex als das erste Material aufweist.

3. Schutz für ein Endoskop nach Anspruch 2, **dadurch gekennzeichnet, dass** das Material, das zum Herstellen des Kerns des zylinderrohrförmigen Teils (8) verwendet wird, ausgewählt ist einer Gruppe umfassend Polystyrole und PMMA (Polymethylmethacrylat).

4. Schutz für ein Endoskop nach Anspruch 2 oder 3, **dadurch gekennzeichnet, dass** das Material, das zum Überdecken des Kerns verwendet wird, ausgewählt ist aus der Gruppe umfassend PMMA (Polymethylmethacrylat) und Fluorpolymere.

5. Schutz für ein Endoskop nach einem der Ansprüche 1 bis 4, **dadurch gekennzeichnet, dass** die Einrichtungen, die ein Streuen und/oder Ausrichten des Lichts gestatten, einen Diffusionssteg (14) und eine Winkelkorrekturlinse (16) enthalten.

6. Schutz für ein Endoskop nach Anspruch 5, **dadurch gekennzeichnet, dass** der Diffusionssteg (14) ein rohrförmiges Teil ist, das eine flache Querseite aufweist, die dazu bestimmt ist, am distalen Ende (12) des zylinderrohrförmigen Teils (8) aufzuliegen, während die der Querseite entgegengesetzte Seite mehrere, gegebenenfalls abgerundete Kanten aufweist, so dass mehrere Prismen (30) entstehen.

7. Schutz für ein Endoskop nach einem der Ansprüche 5 oder 6, **dadurch gekennzeichnet, dass** der Diffusionssteg (14) zwischen der Winkelkorrekturlinse (16) und dem distalen Ende (12) des zylinderrohrförmigen Teils (8) angeordnet ist und dass ein Freiraum zwischen Diffusionssteg (14) und Winkelkorrekturlinse (16) bestehen bleibt, so dass eine Luftlinse (32) zwischen diesen beiden Elementen gebildet ist.

8. Schutz für ein Endoskop nach einem der Ansprüche 1 bis 7, **dadurch gekennzeichnet, dass** der Außendurchmesser seines starren rohrförmigen Teils kleiner als 5 mm ist.

9. Endoskop mit einem invasiven Teil, der ein proximales Ende und ein distales Ende aufweist, **dadurch gekennzeichnet, dass** der invasive Teil einerseits eine starre Stange mit Einrichtungen, die ein Transportieren einer Abbildung von ihrem distalen Teil zu ihrem proximalen Teil gestatten, und andererseits eine Schutzvorrichtung nach einem der Ansprüche 1 bis 8 enthält, und dass das Endoskop ferner einen Lichtauslass aufweist, der gestattet, mittels Beleuchtungseinrichtungen einen ringförmigen Lichtstrahl um das proximale Ende der starren Stange herum zu bilden, sowie Befestigungseinrichtungen zum Aufnahmen der Schutzvorrichtung.

10. Endoskop nach Anspruch 9, **dadurch gekennzeichnet, dass** es aus einem Videoendoskop besteht, und dass es ein Gehäuse (2) enthält, in welchem sich ein Bildsensor (44) und ein dem Bildsensor (44) zugeordnetes Objektiv (46) befinden, und dass der Bildsensor (44) koaxial mit einem konischen Lichtleiter (54) montiert ist, der dem Objektiv (46) so vorgelagert ist, dass das von externen Beleuchtungseinrichtungen stammende Licht geleitet werden kann, um den ringförmigen Lichtstrahl zu bilden.

11. Endoskop nach einem der Ansprüche 9 oder 10, **dadurch gekennzeichnet, dass** die starre Stange aus einem äußeren Metallrohr besteht, in welchem ein optischer Stab angeordnet ist, der dazu bestimmt ist, eine Abbildung von dem distalen Ende der Stange zu ihrem proximalen Ende zu übertragen.

## Claims

1. Endoscope protection device including a sheath with a rigid cylindrical tubular part (8) and an associated flexible sleeve (34),
**characterized in that** the rigid cylindrical tubular part (8) is produced in a material able to transport light, **in that** the rigid cylindrical tubular part (8) is treated to guide light from a light source from its proximal end (10) to its distal end (12), and **in that** the distal end (12) of the cylindrical tubular part (8) includes means (14, 16) for scattering and/or orienting light guided by the cylindrical tubular part (8).

2. Endoscope protection device according to claim 1, **characterized in that** the cylindrical tubular part (8) includes a core made from a first material, and **in that** the core of this cylindrical tubular part has its interior and exterior faces covered with a covering made from a material having a refractive index lower than that of the first material.

3. Endoscope protection device according to claim 2, **characterized in that** the material used to make the core of the cylindrical tubular part (8) is chosen from the group comprising PMMAs (polymethylmethacrylates) and polystyrenes.

4. Endoscope protection device according to claim 2 or claim 3, **characterized in that** the material used to coat the core is chosen from the group comprising PMMA (polymethylmethacrylates) and fluorinated polymers.

5. Endoscope protection device according to any one of claims 1 to 4, **characterized in that** the means for scattering and/or orienting light include a scatter spacer (14) and an angular correction lens (16).

6. Endoscope protection device according to claim 5, **characterized in that** the scatter spacer (14) is a tubular part having a planar transverse face adapted to rest against the distal end (12) of the cylindrical tubular part (8) while the face opposite the transverse face has a number of edges, possibly rounded edges, to form a number of prisms (30).

7. Endoscope protection device according to either of claims 5 or 6, **characterized in that** the scatter spacer (14) is disposed between the angular correction lens (16) and the distal end (12) of the cylindrical tubular part (8) and **in that** there is a gap between the scatter spacer (14) and the angular correction lens (16) so as to form an air lens (32) between those two components.

8. Endoscope protection device according to any one of claims 1 to 7, **characterized in that** the outside diameter of its rigid tubular part is less than 5 mm.

9. Endoscope including an invasive part having a proximal end and a distal end,
**characterized in that** the invasive part includes a rigid stem with means for transporting an image from its distal part to its proximal part and further includes a protection device according to any one of claims 1 to 8 and **in that** the endoscope further includes a light output for forming, with the aid of illumination means, an annular light beam around the proximal end of the rigid stem and fixing means for receiving the protection device.

10. Endoscope according to claim 9, **characterized in that** it is a video endoscope and includes a housing (2) in which there are an image sensor (44) and an optical lens assembly (46) associated with the image sensor (44), **in that** the image sensor (44) is mounted coaxially with a conical light guide (54) disposed forward of the optical lens assembly (46) to direct light from external illumination means to produce the annular light beam.

11. Endoscope according to either of claims 9 or 10, **characterized in that** the rigid stem consists of an external metal tube inside which is disposed an optical rod for transmitting an image from the distal end of the stem to its proximal end.
